# EUROPEAN PATENT APPLICATION

(11) **EP 3 348 551 A1**
(43) Date of publication of application: **18.07.2018**
(21) Application number: 17151604.0
(22) Date of filing: 16.01.2017
(51) Int. Cl.: C07D 307/36, C07D 307/58, C07D 307/60, B01J 27/199

(54) **PROCESS FOR PREPARING AN ORGANIC HETEROCYCLIC COMPOUND**

(71) Applicant: BASF SE, 67056 Ludwigshafen am Rhein (DE)
(72) Inventor: Lejkowski, Michael, 69123 Heidelberg (DE); Lizandara, Carlos, 67056 Ludwigshafen (DE); Kuschel, Andreas, 69123 Heidelberg (DE); Schunk, Stephan A, 69123 Heidelberg (DE); Lieberknecht, Johannes, 69123 Heidelberg (DE); Mueller, Robert, 69123 Heidelberg (DE); Pinkos, Rolf, 67056 Ludwigshafen (DE); Duefert, Alexander, 67056 Ludwigshafen (DE); Huber, Sabine, 67056 Ludwigshafen (DE)
(74) Representative: Herzog, Fiesser & Partner Patentanwälte PartG mbB

(57) **Abstract**

A process for preparing an organic heterocyclic compound having a 5-membered ring structure and comprising an oxygen atom as heteroatom, said process comprising (i) providing a mixture comprising an organic linear C₄ compound; (ii) providing an oxidative cyclization catalyst comprising an oxidic compound comprising molybdenum, vanadium, a alkali metal, phosphorus, and oxygen; (iii) subjecting the mixture provided in (i) to oxidative cyclization conditions comprising contacting the mixture provided in (i) with the oxidative cyclization catalyst provided in (ii). The catalyst is also claimed.

## Description

The present invention relates to a process for preparing an organic heterocyclic compound having a 5-membered ring structure and comprising an oxygen atom as heteroatom. Further, the present invention relates to an oxidic compound comprising molybdenum, vanadium, an alkali metal, phosphorus, and oxygen, and to a process for the preparation of this compound.

Both tetrahydrofuran and butanediol are compounds produced in world-scale amounts and, thus, are of huge commercial interest. According to present considerations, one aims, for example, at producing these compounds from organic C₂ compounds or from organic C₃ compounds wherein organic linear C₄ compound such as 1-butanol and/or crotonaldehyde play a crucial role as intermediates. From these intermediates, furan can be prepared which in turn is suitably converted into tetrahydrofuran and/or butanediol.

Regarding, for example, the synthesis of furan from crotonaldehyde, only a limited number of publications exists regarding both academic papers and patent literature. Ai et al. disclose the production of furan by vapor-phase oxidation of crotonaldehyde using specific heteropolyacids are catalysts; as catalysts, disclosed are a heteropolyacid prepared from 12-molybdophosphoric acid, a MoO₃-P₂O₅ oxide; and a catalyst prepared from 12-molybdophosphoric acid and cesium carbonate. The latter catalyst was found to be the most efficient one. Lu et al. disclose the liquid-phase oxidation of crotonaldehyde to furan by aqueous copper chloride. Tsai et al. disclose an improvement of furan yield in oxidation of crotonaldehyde by modifying 12-molybdophosphoric acid catalysts; as catalysts, derivatives of 12-molybdophosphoric acid are disclosed which derivatives either contain vanadium or tungsten, or which derivatives contain a cation M which is an alkali metal such as Cs, Ag, Ba, Mn, Co, Cu, Ni, Fe or Bi. US 3,600,405 discloses the preparation of furan by oxidation of specified organic compounds in the presence of a catalyst containing Mo and Bi atoms. According to the examples, a catalyst was employed comprising 38.2 % MoO₃ and 61.8 % Bi₂O₃.

In view of the prior art, it was object of the present invention to provide a novel and advantageous process which is industrially applicable. Therefore, the present invention relates to a process for preparing an organic heterocyclic compound having a 5-membered ring structure and comprising an oxygen atom as heteroatom, said process comprising
(i) providing a mixture comprising an organic linear C₄ compound;
(ii) providing an oxidative cyclization catalyst comprising an oxidic compound comprising molybdenum, vanadium, an alkali metal, phosphorus, and oxygen;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

The term "organic heterocyclic compound having a 5-membered ring structure" as used in the context of the present invention relates to a cyclic compound that has atoms of at least two different elements as members of its ring wherein one of these elements is carbon.

As alkali metal, lithium, sodium, potassium, rubidium, cesium, and mixtures of two or more thereof are conceivable. Preferably, the alkali metal comprises cesium. More preferably, the alkali metal comprised in the oxidic compound is cesium. Preferably, the oxidic compound additionally comprises hydrogen (H).

Preferably, at least 99 weight-%, more preferably at least 99.5 weight-%, more preferably at least 99.9, more preferably at least 99.99 weight-% of the oxidic compound consist of molybdenum, vanadium, the alkali metal, phosphorus, oxygen, and hydrogen.

While the oxidic compound can be any type of oxide, it is preferred that the oxidic compound comprises, preferably is, a heteropolyacid. The term "heteropolyacid" as used according to the present invention is a specific family of mixed oxyacids of metals such as molybdenum, tungsten or vanadium and non-metals such as phosphorus, silicon or arsenic. In particular, term "heteropolyacid" as used according to the present invention refers to a class of acid made up of a particular combination of acidic hydrogen atoms and oxygen with certain metals such as molybdenum, tungsten or vanadium, also usually referred to as "the addenda atom", and non-metals from the p-block of the periodic table, such as silicon, phosphorus or arsenic, also usually referred to as "the hetero atom" wherein the metal addenda atoms linked by oxygen atoms form a cluster with the heteroatom inside bonded via oxygen atoms.

More preferably, the oxidic compound is a heteropolyacid and comprises, preferably has, the composition

MoₐV_{b}Cs_{c}P_{d}OₑH_{f}

wherein
a is in the range of from 8 to 13, preferably in the range of from 9 to 12.5, more preferably in the range of from 10.5 to 12;
b is in the range of from 0.07 to 1.3, preferably in the range of from 0.08 to 1.2, more preferably in the range of from 0.09 to 1.1;
c is in the range of from 0.1 to 4, preferably in the range of from 0.5 to 3, more preferably in the range of from 0.75 to 2;
d is in the range of from 0.25 to 1.75, preferably in the range of from 0.5 to 1.5, more preferably in the range of from 0.75 to 1.25;
e is in the range of from 20 to 60, preferably in the range of from 30 to 50, more preferably in the range of from 35 to 45;
f is in the range of from 0.1 to 3, preferably in the range of from 0.5 to 2, more preferably in the range of from 0.75 to 1.25;
wherein more preferably,
b is in the range of from 0.7 to 1.3, more preferably in the range of from 0.8 to 1.2, more preferably in the range of from 0.9 to 1.1.

More preferably, a is in the range of from 8 to 13; b is in the range of from 0.7 to 1.3; c is in the range of from 0.1 to 4; d is in the range of from 0.25 to 1.75; e is in the range of from 20 to 60; f is in the range of from 0.1 to 3. More preferably, a is in the range of from 9 to 12; b is in the range of from 0.8 to 1.2; c is in the range of from 0.5 to 3; d is in the range of from 0.5 to 1.5; e is in the range of from 30 to 50; f is in the range of from 0.5 to 2. More preferably, a is in the range of from 10.5 to 11.5; b is in the range of from 0.9 to 1.1; c is in the range of from 0.75 to 2; d is in the range of from 0.75 to 1.25; e is in the range of from 35 to 45; f is in the range of from 0.75 to 1.25.

Preferably, the oxidation state of the vanadium in the oxidic compound is in the range of from 4 to 5, more preferably in the range of from 4.2 to 4.95, more preferably in the range of from 4.5 to 4.9.

Preferably, the BET specific surface area of the oxidic compound is in the range of from 5 to 100 m²/g, more preferably in the range of from 5 to 90 m²/g, more preferably in the range of from 5 to 50 m²/g, as determined via nitrogen adsorption according to DIN 66131.

According to a first embodiment of the present invention, the oxidative cyclization catalyst consists of the oxidic compound. Regarding this embodiment, the oxidative cyclization catalyst is in the form of a powder, a spray powder, a spray granulate, a shaped body formed by the oxidic compound wherein the shaped body can be a tablet or the like, or a suitably crushed and optionally meshed shaped body.

According to a second embodiment of the present invention, the oxidic compound is supported on a support material, preferably comprising, more preferably being, an oxidic compound. Regarding this embodiment, the oxidative cyclization catalyst comprises, preferably consists of the oxidic compound supported on the support material. Regarding the support material, no specific restrictions exist. Preferably, the support material comprises, more preferably is, one or more of an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum- silicon-titanium mixed oxide, an aluminum- silicon-zirconium mixed oxide, an aluminum-titanium-zirconium mixed oxide, a silicon-titanium-zirconium mixed oxide, an aluminum-silicon-titanium-zirconium mixed oxide, and a mixture of two or more of these oxides. The support material may comprise, preferably be, a molecular sieve, preferably a zeolitic material. With regard to the zeolitic framework types, no specific restrictions exist. Generally, it is conceivable that the zeolitic framework type is one of ABW, ACO, AEI, AEL, AEN, AET, AFG, AFI, AFN, AFO, AFR, AFS, AFT, AFV, AFX, AFY, AHT, ANA, APC, APD, AST, ASV, ATN, ATO, ATS, ATT, ATV, AVL, AWO, AWW, BCT, BEA, BEC, BIK, BOF, BOG, BOZ, BPH, BRE, BSV, CAN, CAS, CDO, CFI, CGF, CGS, CHA, -CHI, -CLO, CON, CSV, CZP, DAC, DDR, DFO, DFT, DOH, DON, EAB, EDI, EEI, EMT, EON, EPI, ERI, ESV, ETR, EUO, *-EWT, EZT, FAR, FAU, FER, FRA, GIS, GIU, GME, GON, GOO, HEU, IFO, IFR, -IFU, IFW, IFY, IHW, IMF, IRN, IRR, -IRY, ISV, ITE, ITG, ITH, *-ITN, ITR, ITT, -ITV, ITW, IWR, IWS, IWV, IWW, JBW, JNT, JOZ, JRY, JSN, JSR, JST, JSW, KFI, LAU, LEV, LIO, -LIT, LOS, LOV, LTA, LTF, LTJ, LTL, LTN, MAR, MAZ, MEI, MEL, MEP, MER, MFI, MFS, MON, MOR, MOZ, *MRE, MSE, MSO, MTF, MTN, MTT, MTW, MVY, MWF, MWW, NAB, NAT, NES, NON, NPO, NPT, NSI, OBW, OFF, OKO, OSI, OSO, OWE, -PAR, PAU, PCR, PHI, PON, POS, PSI, PUN, RHO, -RON, RRO, RSN, RTE, RTH, RUT, RWR, RWY, SAF, SAO, SAS, SAT, SAV, SBE, SBN, SBS, SBT, SEW, SFE, SFF, SFG, SFH, SFN, SFO, SFS, *SFV, SFW, SGT, SIV, SOD, SOF, SOS, SSF, *-SSO, SSY, STF, STI, *STO, STT, STW, -SVR, SVV, SZR, TER, THO, TOL, TON, TSC, TUN, UEI, UFI, UOS, UOV, UOZ, USI, UTL, UWY, VET, VFI, VNI, VSV, WEI, - WEN, YUG, ZON, or a mixed type of two or more thereof. More preferably, the zeolitic material comprises, more preferably is, one or more of zeolitic materials having a framework structure of type MFI, MOR, BEA, and FAU.

Generally, there are no specific restrictions how the oxidative cyclization catalyst provided in (ii) is prepared. Preferably, it is prepared from a mixture, preferably an aqueous mixture, wherein the mixture comprises suitable sources for molybdenum, vanadium, alkali metal, phosphorus, and oxygen wherein a given source may act as source for one or more of molybdenum, vanadium, alkali metal, phosphorus, and oxygen. This mixture, in particular the aqueous mixture, is then preferably subjected to a drying process, preferably a dehydration process, more preferably a freeze-drying process or lyophilization process, said drying process preferably being followed by a calcination process. The overall process may also comprise a shaping process

Preferably, the present invention relates to the process as defined above, wherein the oxidative cyclization catalyst provided in (ii) is obtainable or obtained by a method comprising
(a) preparing an aqueous mixture comprising water and one or more sources for molybdenum, vanadium, alkali metal, phosphorus, and oxygen;
(b) subjecting the mixture preparing in (a) to lyophilization;
(c) preferably subjecting the mixture obtained from (b) to shaping and subsequent crushing, obtaining a lyophilized powder;
(d) subjecting the mixture obtained from (b), preferably the powder obtained from (c), to calcination;
wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄; wherein according to (b), subjecting the mixture to lyophilization preferably comprises cooling the mixture to a temperature in the range of from -50 to -5 °C, more preferably in the range of from -40 to -10 °C, more preferably in the range of from -30 to -15 °C; wherein according to (c), subjecting the mixture to shaping preferably comprises mixing the mixture with a shaping auxiliary, preferably graphite;
wherein according to (d), subjecting to calcination preferably comprises a calcination temperature in the range of from 200 to 350 °C, more preferably in the range of from 250 to 300 °C.

Also preferably, the present invention relates to the process as defined above, wherein providing the oxidative cyclization catalyst according to (ii) comprises
(a) preparing an aqueous mixture comprising water and one or more sources for molybdenum, vanadium, alkali metal, phosphorus, and oxygen;
(b) subjecting the mixture preparing in (a) to lyophilization;
(c) preferably subjecting the mixture obtained from (b) to shaping and subsequent crushing, obtaining a lyophilized powder;
(d) subjecting the mixture obtained from (b), preferably the powder obtained from (c), to calcination;
wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄;
wherein according to (b), subjecting the mixture to lyophilization preferably comprises cooling the mixture to a temperature in the range of from -50 to -5 °C, more preferably in the range of from -40 to -10 °C, more preferably in the range of from -30 to -15 °C; wherein according to (c), subjecting the mixture to shaping preferably comprises mixing the mixture with a shaping auxiliary, preferably graphite;
wherein according to (d), subjecting to calcination preferably comprises a calcination temperature in the range of from 200 to 350 °C, more preferably in the range of from 250 to 300 °C.

Further, the present invention relates to an oxidative cyclization catalyst, obtainable or obtained by a method comprising
(a) preparing an aqueous mixture comprising water and one or more sources for molybdenum, vanadium, alkali metal, phosphorus, and oxygen;
(b) subjecting the mixture preparing in (a) to lyophilization;
(c) preferably subjecting the mixture obtained from (b) to shaping and subsequent crushing, obtaining a lyophilized powder;
(d) subjecting the mixture obtained from (b), preferably the powder obtained from (c), to calcination;
wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄;
wherein according to (b), subjecting the mixture to lyophilization preferably comprises cooling the mixture to a temperature in the range of from -50 to -5 °C, more preferably in the range of from -40 to -10 °C, more preferably in the range of from -30 to -15 °C; wherein according to (c), subjecting the mixture to shaping preferably comprises mixing the mixture with a shaping auxiliary, preferably graphite;
wherein according to (d), subjecting to calcination preferably comprises a calcination temperature in the range of from 200 to 350 °C, more preferably in the range of from 250 to 300 °C.

Regarding the organic linear C₄ compound provided in (i), it is preferred that the compound is comprises, preferably is, one or more of n-butane, 1-butene, 2-butene, 1-butanol, butanal, crotonic acid, crotyl alcohol and crotonaldehyde. More preferably, the organic linear C₄ compound provided in (i) comprises crotonaldehyde. More preferably, the organic linear C₄ compound provided in (i) is crotonaldehyde.

Therefore, the present invention also relates to a process for preparing an organic heterocyclic compound having a 5-membered ring structure and comprising an oxygen atom as heteroatom, said process comprising
(i) providing a mixture comprising crotonaldehyde;
(ii) providing an oxidative cyclization catalyst comprising an oxidic compound comprising molybdenum, vanadium, an alkali metal, phosphorus, and oxygen;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

Further, the present invention also relates to a process for preparing an organic heterocyclic compound having a 5-membered ring structure and comprising an oxygen atom as heteroatom, said process comprising
(i) providing a mixture comprising crotonaldehyde;
(ii) providing an oxidative cyclization catalyst comprising an oxidic compound being a heteropolyacid and comprising, preferably having, the composition

   MoₐV_{b}Cs_{c}P_{d}OₑH_{f}

   wherein the indices a, b, c, d, and e are as defined above;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

Regarding the oxidative cyclization conditions according to (iii), it is preferred that these conditions comprise the presence of molecular oxygen. More preferably, the mixture provided in (i) further comprises molecular oxygen.

Therefore, the present invention also relates to a process for preparing an organic heterocyclic compound having a 5-membered ring structure and comprising an oxygen atom as heteroatom, said process comprising
(i) providing a mixture comprising crotonaldehyde and oxygen;
(ii) providing an oxidative cyclization catalyst comprising an oxidic compound comprising molybdenum, vanadium, an alkali metal, phosphorus, and oxygen;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

Further, the present invention also relates to a process for preparing an organic heterocyclic compound having a 5-membered ring structure and comprising an oxygen atom as heteroatom, said process comprising
(i) providing a mixture comprising crotonaldehyde and oxygen;
(ii) providing an oxidative cyclization catalyst comprising an oxidic compound being a heteropolyacid and comprising, preferably having, the composition

   MoₐV_{b}Cs_{c}P_{d}OₑH_{f}

   wherein the indices a, b, c, d, and e are as defined above;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

Preferably, in the mixture provided in (i), the molar ratio of the molecular oxygen relative to the organic linear C₄ compound is in the range of from 1:1 to 5:1, more preferably in the range of from 2:1 to 4:1, more preferably in the range of from 2.5:1 to 3.5:1.

Preferably, the mixture provided in (i) further comprises water. Therefore, the present invention also relates to a process for preparing an organic heterocyclic compound having a 5-membered ring structure and comprising an oxygen atom as heteroatom, said process comprising
(i) providing a mixture comprising crotonaldehyde, oxygen and water;
(ii) providing an oxidative cyclization catalyst comprising an oxidic compound comprising molybdenum, vanadium, an alkali metal, phosphorus, and oxygen;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

Further, the present invention also relates to a process for preparing an organic heterocyclic compound having a 5-membered ring structure and comprising an oxygen atom as heteroatom, said process comprising
(i) providing a mixture comprising crotonaldehyde, oxygen and water;
(ii) providing an oxidative cyclization catalyst comprising an oxidic compound being a heteropolyacid and comprising, preferably having, the composition

   MoₐV_{b}Cs_{c}P_{d}OₑH_{f}

   wherein the indices a, b, c, d, and e are as defined above;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

Preferably, in the mixture provided in (i), the molar ratio of water relative to the organic linear C₄ oxygenate is in the range of from 0.1:1 to 50:1, more preferably in the range of from 2.5:1 to 35:1, more preferably in the range of from 5:1 to 25:1.

Preferably, the oxidative cyclization conditions according to (iii) comprise the presence of an inert gas. More preferably, the mixture provided in (i) further comprises an inert gas. Preferably, in the mixture provided in (i), the molar ratio of the inert gas relative to the organic linear C₄ compound is in the range of from 10:1 to 90:1, preferably in the range of from 20:1 to 80:1, more preferably in the range of from 30:1 to 70:1. Preferably, the inert gas comprises, preferably is, one or more of nitrogen and argon. More preferably, the inert gas comprises nitrogen.

Generally, it may be conceivable to carry out the cyclization reaction according to (iii) in the liquid phase. Preferably, according to (iii), the mixture comprising the organic linear C₄ compound is contacted in the gas phase with the oxidative cyclization catalyst. The term "gas phase" as used in this context of the present invention refers to the mixture comprising the organic linear C₄ compound in which each component is in the gas phase.

Preferably, the oxidative cyclization conditions according to (iii) comprise a temperature of the mixture in the range of from 240 to 320 °C, more preferably in the range of from 250 to 310 °C, more preferably in the range of from 260 to 290 °C. Preferred temperatures are, for example, in the range of from 260 to 270 °C or in the range of from 270 to 280 °C or in the range of from 280 to 290 °C. Preferably, the temperature of the mixture is in these ranges during the oxidative cyclization reaction.

Preferably, the oxidative cyclization conditions according to (iii) comprise a pressure of the mixture in the range of from 0.25 to 20 bar_{abs}, more preferably in the range of from 0.5 to 10 bar_{abs}, more preferably in the range of from 0.9 to 3 bar_{abs}.

Preferably, the oxidative cyclization conditions according to (iii) comprise a gas hourly space velocity in the range of from 1,500 to 10,000 h⁻¹, more preferably in the range of from 2,000 to 8,000 h⁻¹, more preferably in the range of from 2,400 to 4,000 h⁻¹, wherein the gas hourly space velocity is defined as the volume of gas reaction mixture in ml contacting 1 ml of the oxidative cyclization catalyst within one hour.

Therefore, the present invention also relates to a process for preparing an organic heterocyclic compound having a 5-membered ring structure and comprising an oxygen atom as heteroatom, said process comprising
(i) providing a mixture comprising crotonaldehyde, oxygen, water, and an inert gas;
(ii) providing an oxidative cyclization catalyst comprising an oxidic compound being a heteropolyacid and comprising, preferably having, the composition

   MoₐV_{b}Cs_{c}P_{d}OₑH_{f}

   wherein the indices a, b, c, d, and e are as defined above;
(iii) subjecting the mixture provided in (i) in the gas phase to oxidative cyclization conditions comprising contacting the mixture provided in (i) in the gas phase with the oxidative cyclization catalyst provided in (ii), obtaining a reaction mixture comprising an organic heterocyclic compound having a 5-membered ring structure and comprising an oxygen atom as heteroatom, wherein the oxidative cyclization conditions according to (iii) comprise a temperature of the mixture in the range of from 240 to 320 °C, a pressure of the mixture in the range of from 0.25 to 20 bar_{abs}, and a gas hourly space velocity in the range of from 1,500 to 10,000 h⁻¹.

Further, the present invention also relates to a process for preparing an organic heterocyclic compound having a 5-membered ring structure and comprising an oxygen atom as heteroatom, said process comprising
(i) providing a mixture comprising crotonaldehyde, oxygen, water, and an inert gas;
(ii) providing an oxidative cyclization catalyst comprising an oxidic compound being a heteropolyacid and comprising, preferably having, the composition

   MoₐV_{b}Cs_{c}P_{d}OₑH_{f}

   wherein the indices a, b, c, d, and e are as defined above;
(iii) subjecting the mixture provided in (i) in the gas phase to oxidative cyclization conditions comprising contacting the mixture provided in (i) in the gas phase with the oxidative cyclization catalyst provided in (ii), obtaining a reaction mixture comprising an organic heterocyclic compound having a 5-membered ring structure and comprising an oxygen atom as heteroatom, wherein the oxidative cyclization conditions according to (iii) comprise a temperature of the mixture in the range of from 260 to 290 °C, a pressure of the mixture in the range of from 0.9 to 3 bar_{abs}, and a gas hourly space velocity in the range of from 2,400 to 4,000 h⁻¹.

Preferably, the organic heterocyclic compound having a 5-membered ring structure which is comprised in the reaction mixture obtained from (iii) comprises one or more of maleic anhydride, maleic acid, fumaric acid, furan, and a furanon, preferably 2-furanon, wherein more preferably, the reaction mixture obtained in (iii) comprises maleic anhydride, maleic acid, fumaric acid, furan, and a furanone, preferably 2-furanon. Preferably, the selectivity of the reaction according to (iii) with regard to the maleic anhydride plus the furan plus the furanon is in the range of from 20 to 100 %, preferably in the range of from 40 to 100 %, more preferably in the range of from 60 to 100 %, wherein said selectivity is defined as the molar carbon rate of the compounds maleic anhydride and furan and furanon divided by the difference of the molar carbon rate of the croton aldehyde in the mixture provided in (i) and the molar carbon rate of the croton aldehyde in the mixture obtained from (iii), wherein the term carbon rate of a given compound is defined as . The molar carbon rate is defined as the amount of a compound expressed in mols of carbon atoms included in this compound in a mixture (or in a stream) of this compound per hour.

In case the reaction mixture obtained from (iii) comprises furan, it is possible to recycle said furan, after a suitable separation from the mixture obtained from (iii), back to step (i) of the process of the invention. Thus, the mixture provided in (i) may further comprise furan. For a preferred embodiment described above, the present invention thus relates to a process for preparing an organic heterocyclic compound having a 5-membered ring structure and comprising an oxygen atom as heteroatom, said process comprising
(i) providing a mixture comprising crotonaldehyde, furan, oxygen, water, and an inert gas;
(ii) providing an oxidative cyclization catalyst comprising an oxidic compound being a heteropolyacid and comprising, preferably having, the composition

   MoₐV_{b}Cs_{c}P_{d}OₑH_{f}

   wherein the indices a, b, c, d, and e are as defined above;
(iii) subjecting the mixture provided in (i) in the gas phase to oxidative cyclization conditions comprising contacting the mixture provided in (i) in the gas phase with the oxidative cyclization catalyst provided in (ii), obtaining a reaction mixture comprising maleic anhydride, furan maleic acid, fumaric acid, and a furanone, wherein the oxidative cyclization conditions according to (iii) comprise a temperature of the mixture in the range of from 260 to 290 °C, a pressure of the mixture in the range of from 0.9 to 3 bar_{abs}, and a gas hourly space velocity in the range of from 2,400 to 4,000 h⁻¹;
and preferably separating the furan from the mixture obtained from (iii) and recycling said furan to step (i).

Further, the reaction mixture obtained from (iii) may comprise unreacted organic linear C₄ compound, preferably crotonaldehyde. Yet further, the reaction mixture obtained from (iii) may comprise crotonaldehyde and crotonic acid.

Preferably, the conversion of the organic linear C₄ compound comprised in the mixture provided in (i) and subjected to the reaction in (iii) as starting material is in the range of from 5 to 100 %, preferably in the range of from 10 to 100 %, more preferably in the range of from 20 to 100 %.

Preferably according to the present invention, the process further comprises, after (iii),
(iv) subjecting the reaction mixture obtained from (iii) to reductive conditions, preferably comprising contacting the reaction mixture obtained from (iii) with a reduction catalyst in the presence of hydrogen, obtaining a reaction mixture comprising an organic heterocyclic compound having a 5-membered ring structure and comprising an oxygen atom as heteroatom.

Optionally, the reaction mixture obtained from (iv) further comprises an organic linear compound, preferably an organic linear C₄ compound, which comprises an oxygen atom as heteroatom. The term "comprises an oxygen atom" encompasses embodiments according to which the organic linear compound comprises two or more heteroatoms, such as two or more oxygen atoms as heteroatoms. The term "heteroatom" as used in this specific context includes embodiments according to which the heteroatom such as the oxygen atom is part of the linear chain of the compound and/or is part of a substituent of a linear chain of carbon atoms only. Preferably, the organic linear compound comprises, more preferably is, one or more of a butanediol, preferably 1,4-butanediol, and 1-butanol. More preferably, the organic linear compound comprises, more preferably is, one or more of a butanediol, more preferably 1,4-butanediol.

Prior to (iv) and after (iii), it is possible that unreacted organic linear C₄ compound comprised in the reaction mixture obtained from (iii) is separated from the reaction mixture obtained from (iii). If, for example, crotonaldehyde and crotonic acid are comprised in the reaction mixture obtained from (iii), it is possible that crotonaldehyde and crotonic acid are separated from the reaction mixture obtained from (iii). No specific restrictions exist how this separation is carried out. Preferably, said separation can be carried out by one or more of distillation and absorption, wherein, for example, the preferably cooled products can be absorbed in water, thereby forming the corresponding acids, or be absorbed in a suitable organic solvent. Preferred organic solvents, in particular for absorbing maleic anhydride and a furanon, include suitable alcohols and esters, preferably methanol, 1-butanol, dibutyl-phthalate, and dialkyl phthalate esters.

No specific restrictions exist concerning the chemical nature of the reduction catalyst employed according to (iv). Preferably, the reduction catalyst according to (iv) comprises Co, Ni, Cu or a noble metal, more preferably one or more of ruthenium, platinum and palladium, the reduction catalyst optionally further comprising rhenium. Preferably, the noble metal and optionally the rhenium are supported on a support material preferably comprising one or more of active carbon, an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum- silicon-titanium mixed oxide, an aluminum- silicon-zirconium mixed oxide, an aluminum-titanium-zirconium mixed oxide, a silicon-titanium-zirconium mixed oxide, an aluminum-silicon-titanium-zirconium mixed oxide, and a mixture of two or more thereof.

No specific restrictions exist concerning the reductive conditions according to (iv). Preferably, the reductive conditions comprise a hydrogen pressure in the range of from 2 to 300 bar_{abs}, more preferably in the range of from 10 to 200 bar_{abs}, more preferably in the range of from 20 to 200 bar_{abs}. Preferably, the reductive conditions comprise a temperature of the reaction mixture during (iv) in the range of from 20 to 300 °C, more preferably in the range of from 35 to 275 °C, more preferably in the range of from 50 to 250 °C. Generally, it may be conceivable to carry out the reduction reaction according to (iv) in the gas phase. Preferably, according to (iv), the mixture is contacted in the liquid phase with the reduction catalyst. The term "gas phase" as used in this context of the present invention refers to the mixture in which each component is in the gas phase.

Preferably, the organic heterocyclic compound having a 5-membered ring structure comprised in the reaction mixture obtained from (iv) comprises, preferably is, tetrahydrofuran.

Preferably, the selectivity of the reaction according to (iv) with regard to the tetrahydrofuran is in the range of from 10 to 100 %, preferably in the range of from 20 to 100 %, more preferably in the range of from 30 to 100 %, wherein said selectivity is defined as the molar carbon rate of the tetrahydrofuran comprised in the mixture obtained from (iv) divided by the difference of the molar carbon rate of the mixture obtained from (iii) subjected to (iv) with respect to the furan, the furanones and the maleic anhydride comprised in said mixture, and the molar carbon rate of the tetrahydrofuran and butanediol in the mixture obtained from (iv).

Further, the reaction mixture obtained from (iv) may further comprise one or more of a butanediol, preferably 1,4-butanediol, and 1-butanol. Preferably, the selectivity of the reaction according to (iv) with regard to a butanediol, preferably 1,4-butanediol, is at most 50 %, preferably at most 40 %, more preferably at most 30 %, wherein said selectivity is defined as the molar carbon rate of the butanediol, preferably 1,4-butanediol, comprised in the mixture obtained from (iv) divided by the difference of the molar carbon rate of the mixture obtained from (iii) subjected to (iv) with respect to the furan, the furanones and the maleic anhydride comprised in said mixture, and the molar carbon rate of the tetrahydrofuran and the butanediol, preferably 1,4-butanediol in the mixture obtained from (iv).

Generally, in the event that the mixture obtained from (iv) comprises a butanediol, it is possible to suitably separate the butanediol from the tetrahydrofuran. For this separation, every suitable method can be applied, for example separation by distillation. If, for example, the mixture obtained from (iv) comprises a butanediol, maleic anhydride and a furanon, it is preferred that two distillations are carried out wherein it is preferred that in a first distillation unit, the butanediol and tetrahydrofuran are separated as an azeotrope from the top of said unit, and said azeotrope is then fed to a second distillation unit where tetrahydrofuran is separated as high boiling compound. It may be preferred that the low boiling mixture is recycled to the first distillation unit. Conceivable separation methods are disclosed in US 9,186,559 and US 5,310,954 which are incorporated herein by reference with regard to said separation methods.

Therefore, the present invention also relates to a process for preparing tetrahydrofuran, said process comprising
(i) providing a mixture comprising crotonaldehyde, oxygen and water;
(ii) providing an oxidative cyclization catalyst comprising an oxidic compound being a heteropolyacid and comprising, preferably having, the composition

   MoₐV_{b}Cs_{c}P_{d}OₑH_{f}

   wherein the indices a, b, c, d, and e are as defined above;
(iii) subjecting the mixture provided in (i) to oxidative cyclization conditions comprising contacting the mixture provided in (i) with the oxidative cyclization catalyst provided in (ii), obtaining a reaction mixture comprising one or more of maleic anhydride, furan, maleic acid, fumaric acid and a furanone, preferably 2-furanone;
(iv) subjecting the reaction mixture obtained from (iii) to reductive conditions comprising contacting the reaction mixture obtained from (iii) with a reduction catalyst in the presence of hydrogen, obtaining a reaction mixture comprising the tetrahydrofuran.

The mixture provided according to (i) can be provided in any suitable manner. With regard to the organic linear C₄ compound comprised in the mixture provided according to (i), it is preferred according to a first embodiment of the present invention that the organic linear C₄ compound, preferably crotonaldehyde, comprised in the mixture provided in (i) is suitably prepared from one or more organic C₂ compounds, preferably comprising one or more of ethanol, acetaldehyde, ethylene and acetic acid. With regard to ethanol, it is preferred that the ethanol is condensed by oxidation to acetaldehyde, preferably carried out in the gas phase. With regard to acetaldehyde, it is preferred that it is condensed in the gas phase. With regard to ethylene, it is preferred that it is converted into the corresponding C₄ compound in the liquid phase. It is conceivable that in a first stage, the ethylene is converted to acetaldehyde and in a second stage, the acetaldehyde is converted to the C₄ compound. With regard to acetic acid, it is preferred that it is converted in the gas phase.

According to a second embodiment of the present invention, it is preferred that the organic linear C₄ compound, preferably crotonaldehyde, comprised in the mixture provided in (i) is suitably prepared from one or more organic C₃ compounds, preferably comprising one or more of propane, propene, propanol and propanal, and one or more organic C₁ compounds, preferably comprising one or more of propene, carbon monoxide, carbon dioxide, methanol and formaldehyde, more preferably comprising propene. With regard to propene, it is preferred that it is converted to the C₄ compound in a reaction with carbon monoxide and hydrogen. According to a third embodiment of the present invention, it is preferred that the organic linear C₄ compound, preferably crotonaldehyde, comprised in the mixture provided in (i), is suitably prepared from one or more organic C₃ compounds, preferably comprising one or more of propane, propene, propanol and propanal, and one or more organic C₁ compounds, preferably comprising one or more of propene, carbon monoxide, carbon dioxide, methanol and formaldehyde, more preferably comprising propene.

### Oxidic compound

Yet further, the present invention relates to an oxidic compound comprising molybdenum, vanadium, an alkali metal, phosphorus, and oxygen, wherein at least 99 weight-%, preferably at least 99.5 weight-%, more preferably at least 99.9 weight-%, more preferably at least 99.99 weight-% of the oxidic compound consist of molybdenum, vanadium, the alkali metal, phosphorus, oxygen, and hydrogen.

While the oxidic compound can be any type of oxide, it is preferred that the oxidic compound comprises, preferably is, a heteropolyacid. The term "heteropolyacid" as used according to the present invention is a specific family of mixed oxyacids of metals such as molybdenum, tungsten or vanadium and non-metals such as phosphorus, silicon or arsenic. In particular, term "heteropolyacid" as used according to the present invention refers to a class of acid made up of a particular combination of acidic hydrogen atoms and oxygen with certain metals such as molybdenum, tungsten or vanadium, also usually referred to as "the addenda atom", and non-metals from the p-block of the periodic table, such as silicon, phosphorus or arsenic, also usually referred to as "the hetero atom" wherein the metal addenda atoms linked by oxygen atoms form a cluster with the heteroatom inside bonded via oxygen atoms.

More preferably, the oxidic compound is a heteropolyacid and comprises, preferably has, the composition

MoₐV_{b}Cs_{c}P_{d}OₑH_{f}

wherein
a is in the range of from 8 to 13, preferably in the range of from 9 to 12.5, more preferably in the range of from 10.5 to 12;
b is in the range of from 0.07 to 1.3, preferably in the range of from 0.08 to 1.2, more preferably in the range of from 0.09 to 1.1;
c is in the range of from 0.1 to 4, preferably in the range of from 0.5 to 3, more preferably in the range of from 0.75 to 2;
d is in the range of from 0.25 to 1.75, preferably in the range of from 0.5 to 1.5, more preferably in the range of from 0.75 to 1.25;
e is in the range of from 20 to 60, preferably in the range of from 30 to 50, more preferably in the range of from 35 to 45;
f is in the range of from 0.1 to 3, preferably in the range of from 0.5 to 2, more preferably in the range of from 0.75 to 1.25;
wherein more preferably,
b is in the range of from 0.7 to 1.3, more preferably in the range of from 0.8 to 1.2, more preferably in the range of from 0.9 to 1.1.

More preferably, a is in the range of from 8 to 13; b is in the range of from 0.7 to 1.3; c is in the range of from 0.1 to 4; d is in the range of from 0.25 to 1.75; e is in the range of from 20 to 60; f is in the range of from 0.1 to 3. More preferably, a is in the range of from 9 to 12; b is in the range of from 0.8 to 1.2; c is in the range of from 0.5 to 3; d is in the range of from 0.5 to 1.5; e is in the range of from 30 to 50; f is in the range of from 0.5 to 2. More preferably, a is in the range of from 10.5 to 11.5; b is in the range of from 0.9 to 1.1; c is in the range of from 0.75 to 2; d is in the range of from 0.75 to 1.25; e is in the range of from 35 to 45; f is in the range of from 0.75 to 1.25.

Preferably, the oxidation state of the vanadium in the oxidic compound is in the range of from 4 to 5, more preferably in the range of from 4.2 to 4.95, more preferably in the range of from 4.5 to 4.9.

Preferably, the BET specific surface area of the oxidic compound is in the range of from 5 to 100 m²/g, more preferably in the range of from 5 to 90 m²/g, more preferably in the range of from 5 to 50 m²/g, as determined via nitrogen adsorption according to DIN 66131.

According to a first embodiment of the present invention, the oxidative cyclization catalyst consists of the oxidic compound. Regarding this embodiment, the oxidative cyclization catalyst is in the form of a powder, a spray powder, a spray granulate, a shaped body formed by the oxidic compound wherein the shaped body can be a tablet or the like, or a suitably crushed and optionally meshed shaped body.

According to a second embodiment of the present invention, the oxidic compound is supported on a support material, preferably comprising, more preferably being, an oxidic compound. Regarding this embodiment, the oxidative cyclization catalyst comprises, preferably consists of the oxidic compound supported on the support material. Regarding the support material, no specific restrictions exist. Preferably, the support material comprises, more preferably is, one or more of an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum- silicon-titanium mixed oxide, an aluminum- silicon-zirconium mixed oxide, an aluminum-titanium-zirconium mixed oxide, a silicon-titanium-zirconium mixed oxide, an aluminum-silicon-titanium-zirconium mixed oxide, and a mixture of two or more of these oxides. The support material may comprise, preferably be, a molecular sieve, preferably a zeolitic material. With regard to the zeolitic framework types, no specific restrictions exist. Generally, it is conceivable that the zeolitic framework type is one of ABW, ACO, AEI, AEL, AEN, AET, AFG, AFI, AFN, AFO, AFR, AFS, AFT, AFV, AFX, AFY, AHT, ANA, APC, APD, AST, ASV, ATN, ATO, ATS, ATT, ATV, AVL, AWO, AWW, BCT, BEA, BEC, BIK, BOF, BOG, BOZ, BPH, BRE, BSV, CAN, CAS, CDO, CFI, CGF, CGS, CHA, -CHI, -CLO, CON, CSV, CZP, DAC, DDR, DFO, DFT, DOH, DON, EAB, EDI, EEI, EMT, EON, EPI, ERI, ESV, ETR, EUO, *-EWT, EZT, FAR, FAU, FER, FRA, GIS, GIU, GME, GON, GOO, HEU, IFO, IFR, -IFU, IFW, IFY, IHW, IMF, IRN, IRR, -IRY, ISV, ITE, ITG, ITH, *-ITN, ITR, ITT, -ITV, ITW, IWR, IWS, IWV, IWW, JBW, JNT, JOZ, JRY, JSN, JSR, JST, JSW, KFI, LAU, LEV, LIO, -LIT, LOS, LOV, LTA, LTF, LTJ, LTL, LTN, MAR, MAZ, MEI, MEL, MEP, MER, MFI, MFS, MON, MOR, MOZ, *MRE, MSE, MSO, MTF, MTN, MTT, MTW, MVY, MWF, MWW, NAB, NAT, NES, NON, NPO, NPT, NSI, OBW, OFF, OKO, OSI, OSO, OWE, -PAR, PAU, PCR, PHI, PON, POS, PSI, PUN, RHO, -RON, RRO, RSN, RTE, RTH, RUT, RWR, RWY, SAF, SAO, SAS, SAT, SAV, SBE, SBN, SBS, SBT, SEW, SFE, SFF, SFG, SFH, SFN, SFO, SFS, *SFV, SFW, SGT, SIV, SOD, SOF, SOS, SSF, *-SSO, SSY, STF, STI, *STO, STT, STW, -SVR, SVV, SZR, TER, THO, TOL, TON, TSC, TUN, UEI, UFI, UOS, UOV, UOZ, USI, UTL, UWY, VET, VFI, VNI, VSV, WEI, - WEN, YUG, ZON, or a mixed type of two or more thereof. More preferably, the zeolitic material comprises, more preferably is, one or more of zeolitic materials having a framework structure of type MFI, MOR, BEA, and FAU.

Generally, the oxidative cyclization catalyst can be prepared by any conceivable method. Regarding a preferred method, reference is made to the respective paragraphs herein.

The present invention is further illustrated by the following embodiments and combinations of embodiments as indicated by the respective dependencies and back-references. In particular, it is noted that if a range of embodiments is mentioned, for example in the context of a term such as "The process of any one of embodiments 1 to 4", every embodiment in this range is meant to be disclosed for the skilled person, i.e. the wording of this term is to be understood by the skilled person as being synonymous to "The process of any one of embodiments 1, 2, 3, and 4".
1. A process, preferably a continuous process, for preparing an organic heterocyclic compound having a 5-membered ring structure and comprising an oxygen atom as heteroatom, said process comprising
   (i) providing a mixture comprising an organic linear C₄ compound;
   (ii) providing an oxidative cyclization catalyst comprising an oxidic compound comprising molybdenum, vanadium, an alkali metal, phosphorus, and oxygen;
   (iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.
2. The process of embodiment 1, wherein the alkali metal comprised in the oxidic compound comprises, preferably is, cesium.
3. The process of embodiment 1 or 2, wherein the oxidic compound additionally comprises hydrogen.
4. The process of any one of embodiments 1 to 3, wherein at least 99 weight-%, preferably at least 99.5 weight-%, more preferably at least 99.9, more preferably at least 99.99 weight-% of the oxidic compound consist of molybdenum, vanadium, the alkali metal, phosphorus, oxygen, and hydrogen.
5. The process of any one of embodiments 1 to 4, wherein the oxidic compound is a heteropolyacid and comprises, preferably has, the composition

   MoₐV_{b}Cs_{c}P_{d}OₑH_{f}

   wherein
   a is in the range of from 8 to 13, preferably in the range of from 9 to 12.5, more preferably in the range of from 10.5 to 12;
   b is in the range of from 0.07 to 1.3, preferably in the range of from 0.08 to 1.2, more preferably in the range of from 0.09 to 1.1;
   c is in the range of from 0.1 to 4, preferably in the range of from 0.5 to 3, more preferably in the range of from 0.75 to 2;
   d is in the range of from 0.25 to 1.75, preferably in the range of from 0.5 to 1.5, more preferably in the range of from 0.75 to 1.25;
   e is in the range of from 20 to 60, preferably in the range of from 30 to 50, more preferably in the range of from 35 to 45;
   f is in the range of from 0.1 to 3, preferably in the range of from 0.5 to 2, more preferably in the range of from 0.75 to 1.25,
   wherein more preferably,
   b is in the range of from 0.7 to 1.3, more preferably in the range of from 0.8 to 1.2, more preferably in the range of from 0.9 to 1.1.
6. The process of embodiment 5, wherein a is in the range of from 10.5 to 12; b is in the range of from 0.9 to 1.1; c is in the range of from 0.75 to 2; d is in the range of from 0.75 to 1.25; e is in the range of from 35 to 45; f is in the range of from 0.75 to 1.25.
7. The process of any one of embodiments 1 to 6, wherein in the oxidic compound, the oxidation state of the vanadium is in the range of from 4 to 5, preferably in the range of from 4.2 to 4.95, more preferably in the range of from 4.5 to 4.9.
8. The process of any one of embodiments 1 to 7, wherein the BET specific surface area of the oxidic compound is in the range of from 5 to 100 m²/g, preferably in the range of from 5 to 90 m²/g, more preferably in the range of from 5 to 50 m²/g, as determined via nitrogen adsorption according to DIN 66131.
9. The process of any one of embodiments 1 to 8, wherein the oxidative cyclization catalyst consists of the oxidic compound.
10. The process of any one of embodiments 1 to 9, wherein the oxidic compound is supported on a support material, preferably comprising, more preferably being, an oxidic compound.
11. The process of embodiment 10, wherein the support material comprises, preferably is, one or more of an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum- silicon-titanium mixed oxide, an aluminum- silicon-zirconium mixed oxide, an aluminum-titanium-zirconium mixed oxide, a silicon-titanium-zirconium mixed oxide, an aluminum-silicon-titanium-zirconium mixed oxide, and a mixture of two or more of these oxides.
12. The process of embodiment 10 or 11, wherein the support material comprises, preferably is, a molecular sieve, preferably a zeolitic material, wherein the zeolite preferably comprises, more preferably is, one or more of zeolitic materials having a framework structure of type MFI, MOR, BEA, and FAU.
13. The process of any one of embodiments 10 to 12, wherein the oxidative cyclization catalyst consists of the oxidic compound supported on the support material.
14. The process of any one of embodiments 1 to 13, wherein the oxidative cyclization catalyst provided in (ii) is obtainable or obtained by a method comprising
   (a) preparing an aqueous mixture comprising water and one or more sources for molybdenum, vanadium, alkali metal, phosphorus, and oxygen;
   (b) subjecting the mixture preparing in (a) to lyophilization;
   (c) preferably subjecting the mixture obtained from (b) to shaping and subsequent crushing, obtaining a lyophilized powder;
   (d) subjecting the mixture obtained from (b), preferably the powder obtained from (c), to calcination;
   wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄;
   wherein according to (b), subjecting the mixture to lyophilization preferably comprises cooling the mixture to a temperature in the range of from -50 to -5 °C, more preferably in the range of from -40 to -10 °C, more preferably in the range of from - 30 to -15 °C;
   wherein according to (c), subjecting the mixture to shaping preferably comprises mixing the mixture with a shaping auxiliary, preferably graphite;
   wherein according to (d), subjecting to calcination preferably comprises a calcination temperature in the range of from 200 to 350 °C, more preferably in the range of from 250 to 300 °C.
15. The process of any one of embodiments 1 to 13, wherein providing the oxidative cyclization catalyst according to (ii) comprises
   (a) preparing an aqueous mixture comprising water and one or more sources for molybdenum, vanadium, alkali metal, phosphorus, and oxygen;
   (b) subjecting the mixture preparing in (a) to lyophilization;
   (c) preferably subjecting the mixture obtained from (b) to shaping and subsequent crushing, obtaining a lyophilized powder;
   (d) subjecting the mixture obtained from (b), preferably the powder obtained from (c), to calcination;
   wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄;
   wherein according to (b), subjecting the mixture to lyophilization preferably comprises cooling the mixture to a temperature in the range of from -50 to -5 °C, more preferably in the range of from -40 to -10 °C, more preferably in the range of from -30 to -15 °C;
   wherein according to (c), subjecting the mixture to shaping preferably comprises mixing the mixture with a shaping auxiliary, preferably graphite;
   wherein according to (d), subjecting to calcination preferably comprises a calcination temperature in the range of from 200 to 350 °C, more preferably in the range of from 250 to 300 °C.
16. The process of any one of embodiments 1 to 15, wherein the organic linear C₄ compound comprises, preferably is, one or more of n-butane, 1-butene, 2-butene, 1-butanol, butanal, crotonic acid, crotyl alcohol and crotonaldehyde.
17. The process of any one of embodiments 1 to 16, wherein the linear C₄ molecule is crotonaldehyde.
18. The process of any one of embodiments 1 to 17, wherein the oxidative cyclization conditions according to (iii) comprise the presence of molecular oxygen and the mixture provided in (i) further comprises molecular oxygen.
19. The process of embodiment 18, wherein in the mixture provided in (i), the molar ratio of the molecular oxygen relative to the organic linear C₄ compound is in the range of from 1:1 to 5:1, preferably in the range of from 2:1 to 4:1, more preferably in the range of from 2.5:1 to 3.5:1.
20. The process of any one of embodiments 1 to 19, preferably of embodiment 18 or 19, wherein the mixture provided in (i) further comprises water.
21. The process of embodiment 20, wherein in the mixture provided in (i), the molar ratio of water relative to the organic linear C₄ oxygenate is in the range of from 0.1:1 to 50:1, preferably in the range of from 2.5:1 to 35:1, more preferably in the range of from 5:1 to 25:1.
22. The process of any one of embodiments 1 to 21, wherein the oxidative cyclization conditions according to (iii) comprise the presence of an inert gas and the mixture provided in (i) further comprises an inert gas.
23. The process of embodiment 22, wherein in the mixture provided in (i), the molar ratio of the inert gas relative to the organic linear C₄ compound is in the range of from 10:1 to 90:1, preferably in the range of from 20:1 to 80:1, more preferably in the range of from 30:1 to 70:1.
24. The process of embodiment 22 or 23, wherein the inert gas comprises, preferably is, one or more of nitrogen and argon.
25. The process of any one of embodiments 1 to 24, wherein according to (iii), the mixture comprising the organic linear C₄ compound is contacted in gas phase with the oxidative cyclization catalyst.
26. The process of embodiment 25, wherein the oxidative cyclization conditions according to (iii) comprise a temperature of the mixture in the range of from 240 to 320 °C, preferably in the range of from 250 to 310 °C, more preferably in the range of from 260 to 290 °C.
27. The process of embodiment 25 or 26, wherein the oxidative cyclization conditions according to (iii) comprise a pressure of the mixture in the range of from 0.25 to 20 bar_{abs}, preferably in the range of from 0.5 to 10 bar_{abs}, more preferably in the range of from 0.9 to 3 bar_{abs}.
28. The process of any one of embodiments 25 to 27, wherein the oxidative cyclization conditions according to (iii) comprise a gas hourly space velocity in the range of from 1,500 to 10,000 h⁻¹, preferably in the range of from 2,000 to 8,000 h⁻¹, more preferably in the range of from 2,400 to 4,000 h⁻¹, wherein the gas hourly space velocity is defined as the volume of gas reaction mixture in ml contacting 1 ml of the oxidative cyclization catalyst within one hour.
29. The process of any one of embodiments 1 to 28, wherein the organic heterocyclic compound having a 5-membered ring structure comprised in the reaction mixture obtained from (iii) comprises one or more of maleic anhydride, furan, maleic acid, fumaric acid, and a furanone, preferably 2-furanon, wherein preferably, the reaction mixture obtained in (iii) comprises maleic anhydride, furan, and a furanone, preferably 2-furanon.
30. The process of any one of embodiments 1 to 29, preferably embodiment 29, wherein the reaction mixture obtained from (iii) comprises unreacted organic linear C₄ compound, preferably crotonaldehyde.
31. The process of embodiment 30 or 31, wherein the reaction mixture obtained from (iii) comprises crotonaldehyde and crotonic acid.
32. The process of any one of embodiments 1 to 31, further comprising
   (iv) subjecting the reaction mixture obtained from (iii) to reductive conditions, preferably comprising contacting the reaction mixture obtained from (iii) with a reduction catalyst in the presence of hydrogen, obtaining a reaction mixture comprising an organic heterocyclic compound having a 5-membered ring structure and comprising an oxygen atom as heteroatom, and optionally comprising an organic linear compound comprising an oxygen atom as heteroatom.
33. The process of embodiment 32 insofar as embodiment is dependent on embodiment 30 or 31, wherein prior to (iv), the unreacted organic linear C₄ compound, preferably crotonaldehyde, and preferably crotonic acid are separated from the reaction mixture obtained from (iii).
34. The process of embodiment 33, wherein the unreacted organic linear C₄ compound, preferably crotonaldehyde, and preferably crotonic acid are separated from the reaction mixture obtained from (iii) by one or more of absorption and distillation.
35. The process of any one of embodiments 32 to 34, wherein the reduction catalyst according to (iv) comprises a noble metal, preferably one or more of platinum and palladium, the reduction catalyst optionally further comprising rhenium.
36. The process of embodiment 35, wherein the noble metal and optionally the rhenium are supported on a support material preferably comprising one or more of active carbon, an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum- silicon-titanium mixed oxide, an aluminum- silicon-zirconium mixed oxide, an aluminum-titanium-zirconium mixed oxide, a silicon-titanium-zirconium mixed oxide, an aluminum-silicon-titanium-zirconium mixed oxide, and a mixture of two or more thereof.
37. The process of any one of embodiments 32 to 36, wherein the according to (iv), the reductive conditions comprise a hydrogen pressure in the range of from 2 to 300 bar_{abs}, more preferably in the range of from 10 to 200 bar_{abs}, more preferably in the range of from 20 to 200 bar_{abs}.
38. The process of any one of embodiments 32 to 37, wherein the according to (iv), the reductive conditions comprise a temperature of the reaction mixture during (iv) in the range of from 20 to 300 °C, more preferably in the range of from 35 to 275 °C, more preferably in the range of from 50 to 250 °C.
39. The process of any one of embodiments 32 to 38, wherein the organic heterocyclic compound having a 5-membered ring structure comprised in the reaction mixture obtained from (iv) comprises, preferably is, tetrahydrofuran.
40. The process of any one of embodiments 32 to 39, wherein the reaction mixture obtained from (iv) further comprises an organic linear compound comprising an oxygen atom as heteroatom, preferably one or more of a butanediol, preferably 1,4-butanediol, and 1-butanol, wherein more preferably, the organic linear compound comprising an oxygen atom as heteroatom comprises, more preferably is, 1,4-butanediol.
41. The process of any one of embodiments 32 to 40, wherein the organic heterocyclic compound having a 5-membered ring structure comprised in the reaction mixture obtained from (iv) is separated from the reaction mixture obtained from (iv), preferably by distillation.
42. The process of any one of embodiments 1 to 41, wherein the organic linear C₄ compound, preferably crotonaldehyde, comprised in the mixture provided in (i) is prepared from one or more organic C₂ compounds, preferably comprising one or more of ethanol, acetaldehyde, ethylene and acetic acid.
43. The process of any one of embodiments 1 to 41, further comprising preparing the organic linear C₄ compound, preferably crotonaldehyde, comprised in the mixture provided in (i), from one or more organic C₂ compounds, preferably comprising one or more of ethanol, acetaldehyde, ethylene and acetic acid.
44. The process of any one of embodiments 1 to 41, wherein the organic linear C₄ compound, preferably crotonaldehyde, comprised in the mixture provided in (i) is prepared from one or more organic C₃ compounds, preferably comprising one or more of propane, propene, propanol and propanal, and one or more organic C₁ compounds, preferably comprising one or more of propene, carbon monoxide, carbon dioxide, methanol and formaldehyde, more preferably comprising propene.
45. The process of any one of embodiments 1 to 41, further comprising preparing the organic linear C₄ compound, preferably crotonaldehyde, comprised in the mixture provided in (i), from one or more organic C₃ compounds, preferably comprising one or more of propane, propene, propanol and propanal, and one or more organic C₁ compounds, preferably comprising one or more of carbon monoxide, carbon dioxide, methanol and formaldehyde.
46. An oxidic compound comprising molybdenum, vanadium, an alkali metal, phosphorus, and oxygen, wherein at least 99 weight-%, preferably at least 99.5 weight-%, more preferably at least 99.9 weight-%, more preferably at least 99.99 weight-% of the oxidic compound consist of molybdenum, vanadium, the alkali metal, phosphorus, oxygen, and hydrogen.
47. The oxidic compound of embodiment 46, wherein the alkali metal comprises, preferably is, cesium.
48. The oxidic compound of embodiment 46 or 47, being a heteropolyacid and comprising, preferably having, the composition

   MoₐV_{b}Cs_{c}P_{d}OₑH_{f}

   wherein
   a is in the range of from 8 to 13, preferably in the range of from 9 to 12.5, more preferably in the range of from 10.5 to 12;
   b is in the range of from 0.7 to 1.3, preferably in the range of from 0.8 to 1.2, more preferably in the range of from 0.9 to 1.1;
   c is in the range of from 0.1 to 4, preferably in the range of from 0.5 to 3, more preferably in the range of from 0.75 to 2;
   d is in the range of from 0.25 to 1.75, preferably in the range of from 0.5 to 1.5, more preferably in the range of from 0.75 to 1.25;
   e is in the range of from 20 to 60, preferably in the range of from 30 to 50, more preferably in the range of from 35 to 45;
   f is in the range of from 0.1 to 3, preferably in the range of from 0.5 to 2, more preferably in the range of from 0.75 to 1.25.
49. The oxidic compound of embodiment 48, wherein a is in the range of from 10.5 to 11.5; b is in the range of from 0.9 to 1.1; c is in the range of from 0.75 to 2; d is in the range of from 0.75 to 1.25; e is in the range of from 35 to 45; f is in the range of from 0.75 to 1.25.
50. The oxidic compound of any one of embodiments 46 to 49, wherein the oxidation state of the vanadium is in the range of from 4 to 5, preferably in the range of from 4.2 to 4.95, more preferably in the range of from 4.5 to 4.9.
51. The oxidic compound of any one of embodiments 46 to 50, having a BET specific surface area in the range of from 5 to 100 m²/g, preferably in the range of from 5 to 90 m²/g, more preferably in the range of from 5 to 50 m²/g, as determined via nitrogen adsorption according to DIN 66131.
52. The oxidic compound of any one of embodiments 46 to 51, being obtainable or obtained by a method comprising
   (a) preparing an aqueous mixture comprising water and one or more sources for molybdenum, vanadium, alkali metal, phosphorus, and oxygen;
   (b) subjecting the mixture preparing in (a) to lyophilization;
   (c) preferably subjecting the mixture obtained from (b) to shaping and subsequent crushing, obtaining a lyophilized powder;
   (d) subjecting the mixture obtained from (b), preferably the powder obtained from (c), to calcination;
   wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄;
   wherein according to (b), subjecting the mixture to lyophilization preferably comprises cooling the mixture to a temperature in the range of from -50 to -5 °C, more preferably in the range of from -40 to -10 °C, more preferably in the range of from -30 to -15 °C;
   wherein according to (c), subjecting the mixture to shaping preferably comprises mixing the mixture with a shaping auxiliary, preferably graphite;
   wherein according to (d), subjecting to calcination preferably comprises a calcination temperature in the range of from 200 to 350 °C, more preferably in the range of from 250 to 300 °C.
53. The oxidic compound of any one of embodiments 46 to 52, being supported on a support material, preferably comprising, more preferably being, an oxidic compound.
54. The oxidic compound of embodiment 53, wherein the support material comprises, preferably is, one or more of an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum- silicon-titanium mixed oxide, an aluminum- silicon-zirconium mixed oxide, a aluminum-titanium-zirconium mixed oxide, a silicon-titanium-zirconium mixed oxide, an aluminum-silicon-titanium-zirconium mixed oxide, and a mixture of two or more of these oxides.
55. The oxidic compound of embodiment 53 or 54, wherein the support material comprises, preferably is, a molecular sieve, preferably a zeolitic material, wherein the zeolite preferably comprises, more preferably is, one or more of zeolitic materials having a framework structure of type MFI, MOR, BEA, and FAU.
56. Use of an oxidic compound according to any one of embodiments 46 to 55 as a catalytically active component, preferably for the oxidative cyclization of a linear C₄ compound, more preferably for the oxidative cyclization of crotonaldehyde.
57. An oxidative cyclization catalyst, preferably for the oxidative cyclization of crotonaldehyde, comprising the oxidic compound of any one of embodiments 46 to 55.
58. A process for preparing an oxidic compound comprising molybdenum, vanadium, an alkali metal, phosphorus, and oxygen, comprising
   (a) preparing an aqueous mixture comprising water and one or more sources for molybdenum, vanadium, alkali metal, phosphorus, and oxygen;
   (b) subjecting the mixture preparing in (a) to lyophilization;
   (c) preferably subjecting the mixture obtained from (b) to shaping and subsequent crushing, obtaining a lyophilized powder;
   (d) subjecting the mixture obtained from (b), preferably the powder obtained from (c), to calcination;
   wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄;
   wherein according to (b), subjecting the mixture to lyophilization preferably comprises cooling the mixture to a temperature in the range of from -50 to -5 °C, more preferably in the range of from -40 to -10 °C, more preferably in the range of from - 30 to -15 °C;
   wherein according to (c), subjecting the mixture to shaping preferably comprises mixing the mixture with a shaping auxiliary, preferably graphite;
   wherein according to (d), subjecting to calcination preferably comprises a calcination temperature in the range of from 200 to 350 °C, more preferably in the range of from 250 to 300 °C.
59. The process of embodiment 58, wherein the alkali metal comprises, preferably is, cesium.
60. A process for preparing an oxidative cyclization catalyst, preferably for the oxidative cyclization of crotonaldehyde, comprising supporting an oxidic compound according to any one of embodiments 45 to 55 on a support material, preferably comprising, more preferably being, an oxidic compound.

The present invention is further illustrated by the following reference examples, comparative examples, and examples.

### Examples

### Example 1: Preparation of Mo₁₁P₁Cs₂V₁Oₓ

24.749 g H₃PO₄x 12 MoO₃ (Sigma-Aldrich Chemie GmbH), 24.68 mL V₂O₅ in oxalic acid (0.25 mol/L V₂O₅), 4.87g CsNO₃ (Sigma-Aldrich Chemie GmbH) and 0.171 microL H₃PO₄ (6 mol/L) were mixed with 200 mL DI water and the resulting suspension was stirred for 30 min. Then, the suspension was dropped under stirring into liquid nitrogen, and the suspension was shock frozen. Subsequently, the shock frozen material was posed on a pre-cooled metal dish, and freeze dried at -20 °C and 1.03 mbar_{abs} for 96 h.

The freeze dried material was admixed with 3 weight-% graphite and homogenized. The homogenous mixture was the pressed to round tablets (height: 2-3 mm; diameter: 13 mm) applying a press force of 35 kN using a single punch tablet press (XP 1 from Korsch).

The respectively obtained tablets were crushed and sieved to obtain a 500-1000 micrometer fraction. This fraction was heated under air atmosphere to a temperature of 275 °C at a heating rate of 1 K/min and kept at this temperature for 1 h at an air flow rate of 6 L/min. Subsequently, the calcined material was cooled to room temperature.

The obtained calcined material had a BET specific surface area of 31m²/g, determined via nitrogen adsorption according to DIN 66131. The theoretical composition was H₁Mo₁₁P₁Cs₂V₁O₄₀. The oxidation state of vanadium was 4.65.

### Example 2: Oxidative cyclization

Provided was a gas mixture comprising 1.5 volume-% crotonaldehyde, 4.5 volume-% oxygen (O₂), 7.5 volume-% H₂O, 2 volume-% argon, and 85.5 volume-% nitrogen (N₂). This gas mixture was contacted with the calcined material obtained as described in Example 1 above at a gas hourly space velocity of 2,670 h⁻¹ in a fixed-bed tube reactor containing 1 mg of the catalyst. The testing was carried out in a 48-fold screening unit. The reaction temperature, determined with a thermoelement inside the tube reactor, was 270 °C. From the reaction, a reaction mixture was obtained which contained maleic anhydride, 2-furanon, and furan as the main products. The respective product spectrum was analyzed via a GC unit (GC-MS from Agilent Technologies) equipped with FID, MS and TCD detectors. The selectivity of the reaction, defined as the molar carbon rate of the compounds maleic anhydride and furan and furanon in the product stream divided by the difference of the molar carbon rate of croton aldehyde in the feed stream and the molar carbon rate of croton aldehyde in the product stream, was 81 %. The yield of the reaction, defined as the molar carbon rate of the compounds maleic anhydride and furan and furanon divided by the molar carbon rate of croton aldehyde in the feed stream was 24 %.

### Example 3: Reduction

The reaction mixture obtained as described in Example 2 above, after separation from crotonaldehyde by distillation, was subjected to reduction in the liquid phase. At a temperature of 225 °C and a hydrogen pressure of 160 bar_{abs}, the reaction mixture was contacted with a reduction catalyst comprising Re/C:Pd/C 10:1 in autoclave. A reaction mixture was obtained which contained tetrahydrofuran and 1,4-butanediol as the main products (70 % of GC-contents area).

### Example 4: Oxidative cyclization

Provided was a gas mixture comprising 1 volume-% crotonaldehyde, 3 volume-% oxygen (O₂), 22.5 volume-% H₂O, and 73.5 volume-% nitrogen (N₂). This gas mixture was contacted with the calcined material obtained as described in Example 1 above at a gas hourly space velocity of 2,670 h⁻¹ as described in Example 2 above. The reaction temperature was 270 °C. From the reaction, a reaction mixture was obtained which contained maleic anhydride, 2-furanon, and furan as the main products. The selectivity of the reaction, defined as described in Example 2 above, was 63 %. The yield of the reaction, defined as described in Example 2 above, was 49 %.

### Comparative Examples

In the following Table 1, the results of Example 2 above, achieved with a catalyst of the invention, is compared with results obtained with other catalysts using the same conditions as described in Example 2. Very clearly, the inventive catalyst leads to superior results, both for conversion, yield, and selectivity. The preparation of the comparative catalysts is described hereinbelow.

**Table 1**

| Comparative tests | | | |
|---|---|---|---|
| **Catalyst** | **Conversion / % Crotonaldehyde ¹⁾** | **Yield / % C₄ oxygenates ¹⁾** | **Selectivity / % C₄ oxygenates ¹⁾** |
| | | | |
| M0₁₁P₁Cs₂V₁Oₓ | 29.54 | 23.97 | 81.17 |
| Mo₉Sb_{0.95}O_{28.6} ²⁾ | 4.51 | 3.12 | 69.08 |
| Mo₆Sb₄O₂₄ ³⁾ | 1.61 | 0.63 | 39.24 |
| Mo_{7.4}Sb_{2.6}O_{26.1} ³⁾ | 3.44 | 0.9 | 26.25 |
| 0.05Nb@9.36V/11.3P/Q20C | 3.82 | 0.73 | 19.19 |
| | | | |
| 0.1Nb@9.36V/11.3P/Q20C | 4.03 | 1.35 | 33.44 |
| 3.92Nb_2.62P/Q20C | 3.36 | 0.87 | 25.85 |
| 7.5V+3.5Mo+11.3P/Q20C | 8.96 | 4.62 | 51.55 |
| 6.56V+5.3Mo+11.3P/Q20C | 7.96 | 3.67 | 46.17 |
| 9.36V+1.76Mo+11.3P/Q20 | 7.11 | 2.57 | 36.19 |

| | | | |
|---|---|---|---|
| ¹⁾ C₄ oxygenates: maleic acid anhydride, furan and furanone ²⁾ oxidation at 275 °C ³⁾ oxidation at 425 °C | | | |

### Preparation of the catalysts used for the comparative tests shown in Table 1 above:

Mo₉Sb_{0.95}O₂₈, Mo₆Sb₄O₂₄, Mo_{7.4}Sb_{2.6}O_{26.1}

The catalysts Mo₉Sb_{0.95}O₂₈, Mo₆Sb₄O₂₄ and Mo_{7.4}Sb_{2.6}O_{26.1} were prepared as follows: for each 30 g of a given catalyst, the following mixtures (see Table 2 below) were suspended in 150 mL of deionized water in a 1000 mL flask. Each respectively obtained mixture was refluxed under stirring (magnetic stirred, 300 r.p.m.) for 16 h at 100 °C. Finally, the water was removed and the remaining slurry was slowly solidified under drying at 110 °C. This solid was finally crushed to a 500-1000 micrometer fraction. Thereafter, the samples were calcined (heating ramp 1 K/min to 275 °C, at 275 °C for 1 h, than with heating ramp 1 K/min to 425 °C, at 425 °C for 1 h, than cooled to room temperature). Finally, the fine fraction was separated from the target fraction.

**Table 2**

| Preparation of catalysts | | | |
|---|---|---|---|
| | **amount / g** | **Sb₂O₃ / g (Acros Organics BVBA)** | **MoO₃/g Sigma-Aldrich Chemie GmbH** |
| | | | |
| Mo₉Sb_{0.95}O₂₈ | 30 | 12.102 | 17.924 |
| Mo₆Sb₄O₂₄ | 30 | 2.9 | 27.125 |
| Mo_{7.4}Sb_{2.6}O_{26.1} | 30 | 7.88 | 22.145 |

0.05Nb@9.36V/11.3P/Q20C, 0.1 Nb@9.36V/11.3P/Q20C

The catalysts 0.05Nb@9.36V/11.3P/Q20C and 0.1 Nb@9.36V/11.3P/Q20C were prepared as follows: 6 g of the carrier (Cariact Q20C,315-500 micrometer) were impregnated in a first impregnation step (ICW with 100 % water uptake). The solution used for impregnation was prepared from a metal salt solution and water so that the total volume corresponded to the water uptake capability of the carrier. Solution 1 (see Table 3 below) prepared from ammoniumheptamolybdate und ammoniummetavanadate were impregnated three times in order to achieve the required loading. Between each impregnation step the impregnated material was air-dried for 4 h at 80 °C.

**Table 3**

| Preparation of solution | | | |
|---|---|---|---|
| volumina / microL | | | |
| concentration / mol/l | 0.5 | 0.45 | |
| | Nd ammonium oxalate solution | NH₄VO₃ solution | de-ionized water |
| 0.05Nb@9.36V/11.3P/Q20C | 14.4 | 5,443.3 | 542.3 |
| 0.05Nb@9.36V/11.3P/Q20C | 28.7 | 5,443.3 | 528.0 |

After air-drying, the samples were calcined (heating ramp 1 K/min to 500 °C) at 500 °C for 2 h. After cooling to room temperature, the finally obtained fine fraction was separated from the target fraction.
3.92Nb_2.62P/Q20C

The 3.92Nb_2.62P/Q20C catalyst was prepared as follows: 3 g of carrier (Cariact Q20C / 315-500 micrometer) were impregnated in a first impregnation step (ICW with 100 % water uptake). The solution used for the impregnation was prepared from a metal salt solution and water so that the total volume corresponded to the water uptake capability of the carrier (2,463 microliter, 0.5 mol/l Nb ammoniumoxalate solution and 657 microliter de-ionized water). After the first impregnation step, the samples were left for 30 min (aging) and finally freeze dried in liquid nitrogen. Freeze drying was carried out overnight at -10 °C and 2.56 mbar. The second impregnation was carried out with a solution of H₃PO₄ in water (246 microliter 10 mol/l H₃PO₄ solution and 2,874 microliter de-ionized water). After the second impregnation, the samples were left for 30 min (aging) and finally shock-frozen in liquid nitrogen. Thereafter, the samples were calcined (heating ramp 1 K/min to to 500 °C) at 500 °C for 2 h, than cooled to room temperature). Finally, the fine fraction was separated from the target fraction.
7.5V+3.5Mo+11.3P/Q20C, 6.56V+5.3Mo +11.3P/Q20C, 9.36V+1.76Mo+11.3P/Q20C

The 7.5V+3.5Mo+11.3P/Q20C, 6.56V+5.3Mo +11.3P/Q20C and 9.36V+1.76Mo+11.3P/Q20C catalysts were prepared as follows: 3 g of carrier (Cariact Q20C / 315-500 micrometer) were impregnated in a first impregnation step (ICW with 100 % water uptake). The solution used for impregnation was prepared from a metal salt solution and water so that the total volume corresponded to the water uptake capability of the carrier (see Table 4 below). The resulting samples were air dried at 80 °C for 16 h.

**Table 4**

| First impregnation | | | |
|---|---|---|---|
| volumina / microL | | | |
| concentration / mol/l | 1.1 | 2 | |
| | V₂O₅ in oxalic acid | MoO₃ solution | de-ionized water |
| 7.5V + 3.5Mo + 11.3P / Q20C | 2,007.7 | 547.2 | 565.08 |
| 6.56V + 5.3Mo + 11.3P / Q20C | 1,756.1 | 828.6 | 535.26 |
| .36V + 1.76Mo + 11.3P / Q20C | 2,505.6 | 275.2 | 339.23 |

The second impregnation was carried out with a solution of H₃PO₄ in water (see Table 5 below). The resulting samples were air dried at 80 °C for 16 h.

**Table 5**

| Second impregnation | | |
|---|---|---|
| volumina / microL | | |
| concentration / mol/l | 6 | --- |
| | H₃PO₄ solution | de-ionized water |
| 7.5V + 3.5Mo + 11.3P / Q20C | 1,824.3 | 1,295.7 |
| 6.56V + 5.3Mo + 11.3P / Q20C | 1,824.3 | 1,295.7 |
| .36V + 1.76Mo + 11.3P / Q20C | 1,824.3 | 1,295.7 |

Thereafter, the samples were calcined (heating ramp 1 K/min to to 500 °C) at 500 °C for 2 h, than cooled to room temperature). Finally, the fine fraction was separated from the target fraction.

### Cited Literature

- Ai et al., Bull. Chem. Soc. Jpn., vol. 53 (1980) pp 2647-2650
- Lu et al., React. Kinet. Catal. Lett., vol. 64 no. 1 (1998) pp 15-20
- Tsai et al., Bull. Chem. Soc. Jpn., vol. 54 (1981) pp 2103-2106
- US 3,600,405
- US 9,186,559
- US 5,310,954

## Claims

1. A process for preparing an organic heterocyclic compound having a 5-membered ring structure and comprising an oxygen atom as heteroatom, said process comprising
(i) providing a mixture comprising an organic linear C₄ compound;
(ii) providing an oxidative cyclization catalyst comprising an oxidic compound comprising molybdenum, vanadium, an alkali metal, phosphorus, and oxygen;
(iii) subjecting the mixture provided in (i) to oxidative cyclization conditions comprising contacting the mixture provided in (i) with the oxidative cyclization catalyst provided in (ii), obtaining a reaction mixture comprising an organic heterocyclic compound having a 5-membered ring structure and comprising an oxygen atom as heteroatom.

2. The process of claim 1, wherein the alkali metal comprised in the oxidic compound is cesium and wherein the oxidic compound additionally comprises hydrogen.

3. The process of claim 1 or 2, wherein the oxidic compound is a heteropolyacid and comprises, preferably has, the composition
MoₐV_{b}Cs_{c}P_{d}OₑH_{f}
wherein
a is in the range of from 8 to 13, preferably in the range of from 9 to 12.5, more preferably in the range of from 10.5 to 12;
b is in the range of from 0.7 to 1.3, preferably in the range of from 0.8 to 1.2, more preferably in the range of from 0.9 to 1.1;
c is in the range of from 0.1 to 4, preferably in the range of from 0.5 to 3, more preferably in the range of from 0.75 to 2;
d is in the range of from 0.25 to 1.75, preferably in the range of from 0.5 to 1.5, more preferably in the range of from 0.75 to 1.25;
e is in the range of from 20 to 60, preferably in the range of from 30 to 50, more preferably in the range of from 35 to 45;
f is in the range of from 0.1 to 3, preferably in the range of from 0.5 to 2, more preferably in the range of from 0.75 to 1.25.

4. The process of any one of claims 1 to 3, wherein in the oxidic compound, the oxidation state of the vanadium is in the range of from 4 to 5, preferably in the range of from 4.2 to 4.95, more preferably in the range of from 4.5 to 4.9.

5. The process of any one of claims 1 to 4, wherein the oxidic compound is supported on a support material, wherein the support material comprises, preferably is, one or more of an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum-silicon-titanium mixed oxide, an aluminum-silicon-zirconium mixed oxide, an aluminum-titanium-zirconium mixed oxide, a silicon-titanium-zirconium mixed oxide, an aluminum-silicon-titanium-zirconium mixed oxide, and a mixture of two or more of these oxides.

6. The process of any one of claims 1 to 5, wherein the organic linear C₄ compound comprises, preferably is, one or more of n-butane, 1-butene, 2-butene, 1-butanol, butanal, crotonic acid, crotyl alcohol and crotonaldehyde, wherein the linear C₄ molecule is preferably crotonaldehyde.

7. The process of any one of claims 1 to 6, wherein the oxidative cyclization conditions according to (iii) comprise the presence of molecular oxygen and the mixture provided in (i) further comprises molecular oxygen, wherein in the mixture provided in (i), the molar ratio of the molecular oxygen relative to the organic linear C₄ compound is preferably in the range of from 1:1 to 5:1, more preferably in the range of from 2:1 to 4:1, more preferably in the range of from 2.5:1 to 3.5:1.

8. The process of any one of claims 1 to 7, wherein the mixture provided in (i) further comprises water, wherein in the mixture provided in (i), the molar ratio of water relative to the organic linear C₄ oxygenate is preferably in the range of from 0.1:1 to 50:1, more preferably in the range of from 2.5:1 to 35:1, more preferably in the range of from 5:1 to 25:1.

9. The process of any one of claims 1 to 8, wherein the oxidative cyclization conditions according to (iii) comprise the presence of an inert gas, preferably one or more of nitrogen and argon, and the mixture provided in (i) further comprises an inert gas, preferably one or more of nitrogen and argon, wherein in the mixture provided in (i), the molar ratio of the inert gas relative to the organic linear C₄ compound is preferably in the range of from 10:1 to 90:1, more preferably in the range of from 20:1 to 80:1, more preferably in the range of from 30:1 to 70:1.

10. The process of any one of claims 1 to 9, wherein according to (iii), the mixture comprising the organic linear C₄ compound is contacted in gas phase with the oxidative cyclization catalyst.

11. The process of claim 10, wherein the oxidative cyclization conditions according to (iii) comprise a temperature of the mixture in the range of from 240 to 320 °C, preferably in the range of from 250 to 310 °C, more preferably in the range of from 260 to 290 °C and a pressure of the mixture in the range of from 0.25 to 20 bar_{abs}, preferably in the range of from 0.5 to 10 bar_{abs}, more preferably in the range of from 0.9 to 3 bar_{abs}.

12. The process of any one of claims 1 to 11, wherein the organic heterocyclic compound having a 5-membered ring structure comprised in the reaction mixture obtained from (iii) comprises one or more of maleic anhydride, furan, maleic acid, fumaric acid and a furanone, preferably 2-furanon, wherein preferably, the reaction mixture obtained in (iii) comprises maleic anhydride, furan, and a furanone, preferably 2-furanon.

13. The process of any one of claims 1 to 12, further comprising
(iv) subjecting the reaction mixture obtained from (iii) to reductive conditions, preferably comprising contacting the reaction mixture obtained from (iii) with a reduction catalyst in the presence of hydrogen, obtaining a reaction mixture comprising an organic heterocyclic compound having a 5-membered ring structure and comprising an oxygen atom as heteroatom, and optionally comprising an organic linear compound comprising an oxygen atom as heteroatom;
wherein the reduction catalyst according to (iv) preferably comprises a noble metal, preferably one or more of platinum and palladium, the reduction catalyst optionally further comprising rhenium.

14. The process of claim 13, wherein according to (iv), the reductive conditions comprise a hydrogen pressure in the range of from 2 to 300 bar_{abs}, more preferably in the range of from 10 to 200 bar_{abs}, more preferably in the range of from 20 to 200 bar_{abs} and a temperature of the reaction mixture during (iv) in the range of from 20 to 300 °C, preferably in the range of from 35 to 275 °C, more preferably in the range of from 50 to 250 °C.

15. The process of claim 13 or 14, wherein the organic heterocyclic compound having a 5-membered ring structure comprised in the reaction mixture obtained from (iv) comprises, preferably is, tetrahydrofuran, and wherein the organic linear compound comprising an oxygen atom as heteroatom is preferably 1,4-butanediol.

16. An oxidic compound comprising molybdenum, vanadium, an alkali metal, phosphorus, and oxygen, wherein at least 99 weight-%, preferably at least 99.5 weight-%, more preferably at least 99.9 weight-%, more preferably at least 99.99 weight-% of the oxidic compound consist of molybdenum, vanadium, the alkali metal, phosphorus, oxygen, and hydrogen.

17. The oxidic compound of claim 16, wherein the alkali metal comprises, preferably is, cesium.

18. The oxidic compound of claim 16 or 17, being a heteropolyacid and comprising, preferably having, the composition
MoₐV_{b}Cs_{c}P_{d}OₑH_{f}
wherein
a is in the range of from 8 to 13, preferably in the range of from 9 to 12.5, more preferably in the range of from 10.5 to 12;
b is in the range of from 0.7 to 1.3, preferably in the range of from 0.8 to 1.2, more preferably in the range of from 0.9 to 1.1;
c is in the range of from 0.1 to 4, preferably in the range of from 0.5 to 3, more preferably in the range of from 0.75 to 2;
d is in the range of from 0.25 to 1.75, preferably in the range of from 0.5 to 1.5, more preferably in the range of from 0.75 to 1.25;
e is in the range of from 20 to 60, preferably in the range of from 30 to 50, more preferably in the range of from 35 to 45;
f is in the range of from 0.1 to 3, preferably in the range of from 0.5 to 2, more preferably in the range of from 0.75 to 1.25.

19. The oxidic compound of any one of claims 16 to 18, wherein the oxidation state of the vanadium is in the range of from 4 to 5, preferably in the range of from 4.2 to 4.95, more preferably in the range of from 4.5 to 4.9.

20. The oxidic compound of any one of claims 16 to 19, having a BET specific surface area in the range of from 5 to 100 m²/g, preferably in the range of from 5 to 90 m²/g, more preferably in the range of from 5 to 50 m²/g, as determined via nitrogen adsorption according to DIN 66131.

21. The oxidic compound of any one of claims 16 to 20, obtainable or obtained by a method comprising
(a) preparing an aqueous mixture comprising water and one or more sources for molybdenum, vanadium, alkali metal, phosphorus, and oxygen;
(b) subjecting the mixture preparing in (a) to lyophilization;
(c) preferably subjecting the mixture obtained from (b) to shaping and subsequent crushing, obtaining a lyophilized powder;
(d) subjecting the mixture obtained from (b), preferably the powder obtained from (c), to calcination;
wherein according to (a), a source for molybdenum preferably comprises H₃PO₄ x 12 MoO₃, a source for vanadium preferably comprises V₂O₅, a source for the alkali metal preferably comprises CsNO₃, a source for phosphorus preferably comprises H₃PO₄;
wherein according to (b), subjecting the mixture to lyophilization preferably comprises cooling the mixture to a temperature in the range of from -50 to -5 °C, more preferably in the range of from -40 to -10 °C, more preferably in the range of from -30 to -15 °C;
wherein according to (c), subjecting the mixture to shaping preferably comprises mixing the mixture with a shaping auxiliary, preferably graphite;
wherein according to (d), subjecting to calcination preferably comprises a calcination temperature in the range of from 200 to 350 °C, more preferably in the range of from 250 to 300 °C.

22. The oxidic compound of any one of claims 16 to 21, being supported on a support material, preferably comprising, more preferably being, an oxidic compound, more preferably comprises, more preferably being, one or more of an aluminum oxide, a silicon oxide, a titanium oxide, a zirconium oxide, an aluminum-silicon mixed oxide, an aluminum-titanium mixed oxide, an aluminum-zirconium mixed oxide, a silicon-titanium mixed oxide, a silicon-zirconium mixed oxide, a titanium-zirconium mixed oxide, an aluminum- silicon-titanium mixed oxide, an aluminum- silicon-zirconium mixed oxide, a aluminum-titanium-zirconium mixed oxide, a silicon-titanium-zirconium mixed oxide, an aluminum-silicon-titanium-zirconium mixed oxide, and a mixture of two or more of these oxides.

23. Use of an oxidic compound according to any one of claims 16 to 22 as a catalytically active component, preferably for the oxidative cyclization of a linear C₄ compound, more preferably for the oxidative cyclization of crotonaldehyde.

24. An oxidative cyclization catalyst, preferably for the oxidative cyclization of crotonaldehyde, comprising the oxidic compound of any one of claims 16 to 22.

25. A process for preparing an oxidic compound comprising molybdenum, vanadium, an alkali metal, phosphorus, and oxygen, preferably for preparing oxidic compound according to any one of claims 16 to 20, the process comprising
(a) preparing an aqueous mixture comprising water and one or more sources for molybdenum, vanadium, alkali metal, phosphorus, and oxygen;
(b) subjecting the mixture preparing in (a) to lyophilization;
(c) preferably subjecting the mixture obtained from (b) to shaping and subsequent crushing, obtaining a lyophilized powder;
(d) subjecting the mixture obtained from (b), preferably the powder obtained from (c), to calcination;
wherein according to (a), a source for molybdenum preferably comprises H₃PO₄ x 12 MoO₃, a source for vanadium preferably comprises V₂O₅ a source for the alkali metal preferably comprises CsNO₃, a source for phosphorus preferably comprises H₃PO₄;
wherein according to (b), subjecting the mixture to lyophilization preferably comprises cooling the mixture to a temperature in the range of from -50 to -5 °C, more preferably in the range of from -40 to -10 °C, more preferably in the range of from -30 to -15 °C;
wherein according to (c), subjecting the mixture to shaping preferably comprises mixing the mixture with a shaping auxiliary, preferably graphite;
wherein according to (d), subjecting to calcination preferably comprises a calcination temperature in the range of from 200 to 350 °C, more preferably in the range of from 250 to 300 °C.
